# FASCICULE DE BREVET EUROPEEN

(11) **EP 1 739 084 B1**
(45) Date de publication et mention de la délivrance du brevet: **15.09.2010**
(21) Numéro de dépôt: 06116056.0
(22) Date de dépôt: 26.06.2006
(51) Int. Cl.: C07D 295/13, C07C 211/51, A61Q 5/10

(54) **Para-phénylènediamines doubles reliées par un bras de liaison comportant un radical cyclique saturé et utilisation en coloration**
Para-phenylendiamine verbunden über einen Linker der einen gesättigten Ring enthält und Verwendung als Färbemittel
Double para-phenylenediamines joined by a linker comprising a saturated cyclic radical and use thereof as dyeing agents

(30) Priorité: 29.06.2005 FR 0551805
(43) Date de publication de la demande: 03.01.2007
(73) Titulaire: L'Oréal, 75008 Paris (FR)
(72) Inventeur: Sabelle, Stéphane, 75005 Paris (FR); Metais, Eric, 95320 St Leu la Forêt (FR); Radisson, Xavier, 75014 Paris (FR)
(74) Mandataire: Fevrier, Murielle Françoise E.

(56) Documents cités:
- WO-A-01/72686
- WO-A-98/01434
- WO-A-02/055500
- WO-A-03/024917
- SELIGMAN A M ET AL: "SOME CYTOCHEMICAL CORRELATIONS BETWEEN OXIDASE ACTIVITY (CYTOCHROMEAND PEROXIDASE) AND CHEMICAL STRUCTURE OF BIS(PHENYLENEDIAMINES)" HISTOCHEMIE, SPRINGER, BERLIN,, DE, vol. 22, 1970, pages 85-99, XP000926757 ISSN: 0018-2222
- WINKELMANN E ET AL: "Chemotherapeutisch wirksame Nitroverbindungen" ARZNEIMITTEL FORSCHUNG. DRUG RESEARCH, ECV EDITIO CANTOR VERLAG, AULENDORF, DE, vol. 25, no. 5, mai 1975 (1975-05), pages 681-708, XP002122990 ISSN: 0004-4172
- CHEN Y ET AL: "CHEMICAL BEHAVIOR OF N-ARYL NITROGENOXOSQUARIC ACID IN ALCOHOLS" SICHUAN DAXUE XUEBA, ZIRAN KEXUE - ACTA SCIETIARUM NATURALIUM UNIVERSITATIS SZECHUANENSIS, SSU CH'UAN TA HSUEH, CHENG-TU, CN, vol. 35, no. 1, février 1998 (1998-02), pages 141-143, XP001094496 ISSN: 0490-6756
- KOLSAKER ET AL.: "Ozonation of p-nitro-N,N-dimethylaniline" ADVANCES IN CHEMISTRY SERIES, vol. 112, 1972, pages 101-113, XP001246465

## Description

La présente demande concerne une nouvelle famille de para-phénylènediamines doubles reliées par un bras de liaison comportant un radical cyclique saturé et leur utilisation pour la coloration des fibres kératiniques.

Il est connu de teindre les fibres kératiniques et en particulier les cheveux humains avec des compositions tinctoriales contenant des précurseurs de colorant d'oxydation, appelés généralement bases d'oxydation, tels que des ortho- ou para-phénylènediamines, des ortho- ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diaminobenzènes aromatiques, les méta-aminophénols, les méta-diphénols et certains composés hétérocycliques tels que des composés indoliques et pyridiniques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (c'est-à-dire abîmée) entre sa pointe et sa racine.

Le but de la présente invention est de fournir de nouvelles bases d'oxydation capables de colorer les fibres kératiniques avec des nuances variées, puissantes, esthétiques et peu sélectives ainsi que des colorations résistantes aux diverses agressions que peuvent subir les fibres telles que la lumière, la sueur et les shampoings.

Ce but est atteint avec la présente invention qui a pour objet une nouvelle famille de para-phénylènediamine double de formule (I) suivante ainsi que les sels d'addition correspondants: 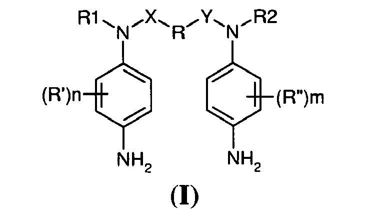 dans laquelle :
- R représente un cycle saturé de 4 à 7 chaînons, contenant éventuellement un ou plusieurs atomes d'azote ou d'oxygène ; ce cycle pouvant être éventuellement substitué notamment par un ou plusieurs groupements alkyles en C₁-C₄,
- R₁ et R2 représentent, indépendamment l'un de l'autre,
   - un atome d'hydrogène,
   - un radical alkyle en C₁-C₆linéaire ou ramifié,
   - un radical alkyle en C₁-C₆ linéaire ou ramifié substitué par un ou plusieurs radicaux hydroxy, alkoxy en C₁-C₄, amino, monoalkyl amino en C₁-C₄, dialkylamino en C₁-C₄,
- X et Y représentent, indépendamment l'un de l'autre, un radical alkylène en C₁-C₁₀ linéaire ou ramifié ou une liaison covalente,
- R' et R" représentent, indépendamment l'un de l'autre,
   - un radical alkyle en C₁-C₆,
   - un radical alcoxy en C₁-C₆,
   - un radical hydroxy-alcoxy en C₁-C₆,
   - un radical alcoxy(C₁-C₆)alkyle en C₁-C₆,
   - un radical mono ou poly-hydroxy alkyle en C₁-C₆,
      n et m représentent, indépendamment l'un de l'autre, un entier compris entre 0 et 4
      à l'exception des composés suivants :

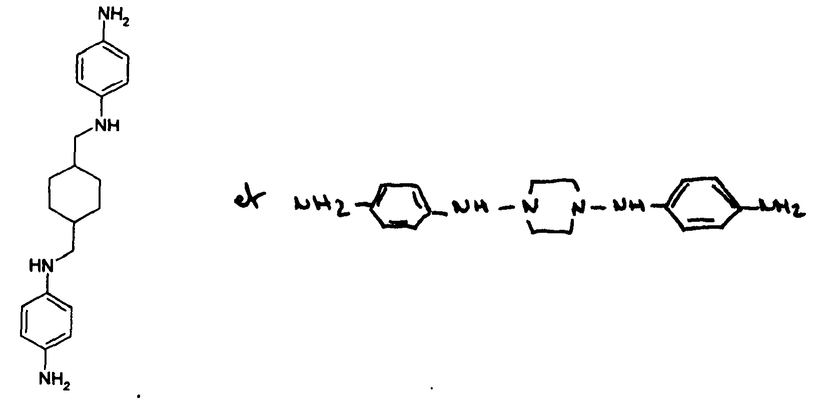

La composition de la présente invention permet en particulier d'obtenir une coloration de fibres kératiniques très puissante, peu sélective et tenace, en particulier à la lumière.

Un autre objet de l'invention concerne des compositions pour la coloration des fibres kératiniques, en particulier des fibres kératiniques humaines telles que les cheveux, comprenant au moins une para-phénylènediamine de formule (I).

L'invention a aussi pour objet un procédé de coloration mettant en oeuvre cette composition, l'utilisation de la composition selon la présente invention pour la coloration des fibres kératiniques, en particulier les fibres kératiniques humaines telles que les cheveux et un dispositif à plusieurs compartiments ou "kit" de coloration.

A titre d'exemple, on peut citer les paraphénylènediamines suivantes :

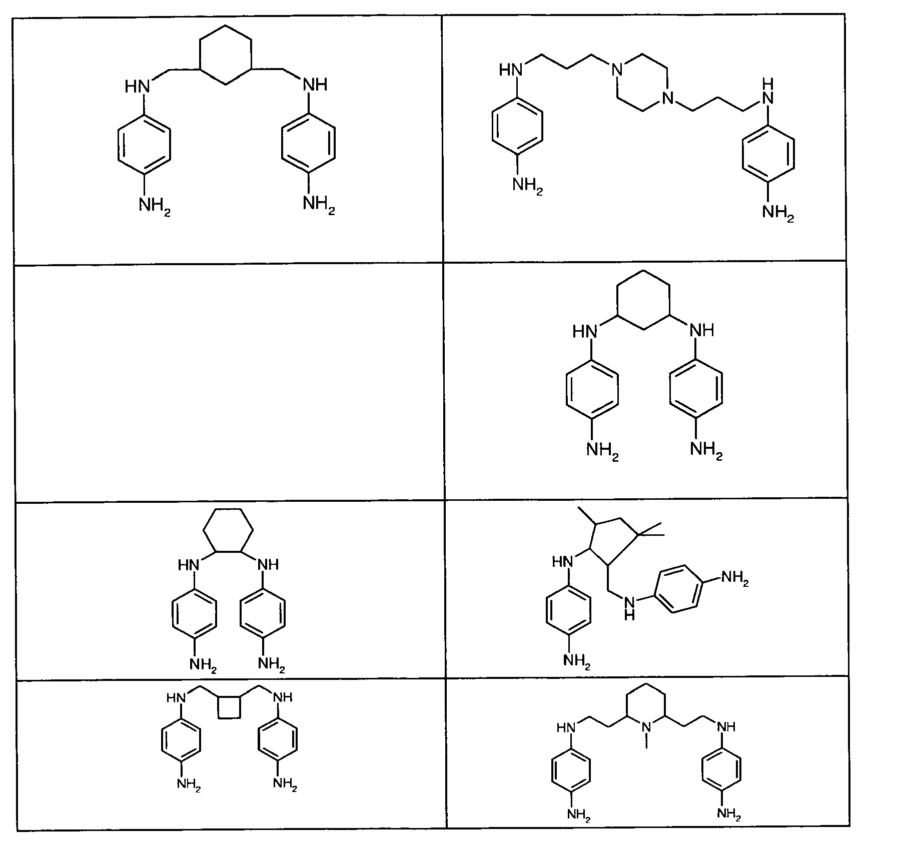

Selon un mode de réalisation particulier, les para-phénylènediamines de formule (I) sont telles que R est un radical cyclique saturé contenant éventuellement un atome d'azote. A titre de substituant de R, on peut citer les groupements alkyles en C₁-C₄. R₁ et R₂ représentent de préférence indépendamment un atome d'hydrogène ou un groupe alkyle en C₁-C₄ pouvant être substitué. X et Y représentent indépendamment de préférence un radical alkylène en C₁-C₃ ou une liaison covalente. n et m sont de préférence égal à 0 ou 1.

D'une manière générale, les sels d'addition utilisables sont notamment choisis parmi les sels d'addition avec un acide, tels que l'acide chlorhydrique, l'acide bromhydrique, l'acide sulfurique, l'acide citrique, l'acide succinique, l'acide tartrique, l'acide lactique, l'acide para-toluènesulfonique, l'acide benzènesulfonique, l'acide phosphorique et l'acide acétique.

Ils peuvent aussi être sous forme de solvates par exemple un hydrate ou un solvate d'alcool linéaire ou ramifié tel que l'éthanol ou l'isopropanol.

Les para-phénylènediamines de formule (I) selon la présente demande peuvent être préparés suivant une méthode de synthèse classique. On pourra par exemple se reporter à la demande de brevet DE 10 144226A.

A titre d'illustration, les para-phénylènediamines de formule (I) peuvent être synthétisés selon le schéma réactionnel suivant :

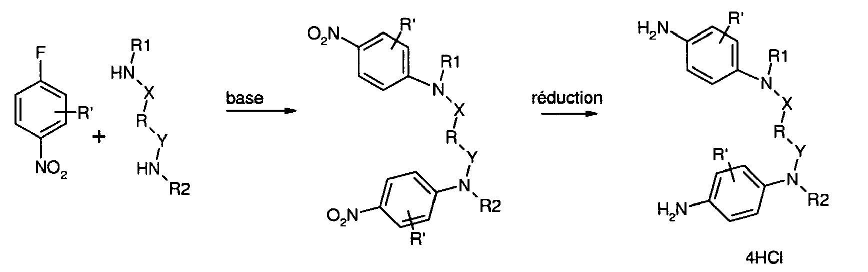

La première étape de la synthèse est une substitution nucléophile d'une diamine sur un dérivé de para-fluoro-nitrobenzène, étape inspirée des publications Synthesis 1990 (12), 1147-1148 et Synth. Commun. 1990, 20 (22), 3537-3545. La deuxième étape est une étape de réduction classique, pouvant être par exemple une réaction d'hydrogénation par catalyse hétérogène en présence de Pd/C, Pd(II)/C, Ni de Raney ou encore une réaction de réduction par un métal, par exemple par du zinc, fer, étain... (Advanced Organic Chemistry, 4th edition, 1992, J. MARCH, WILEY Interscience ; Reduction in Organic Chemistry, M. Hudlicky, 1983, Ellis Honwood séries Chemical Science).

La présente demande concerne aussi les composés nitrés de formule (II) suivante qui peuvent être utilisés pour l'obtention des para-phénylènediamines de formule (I) : 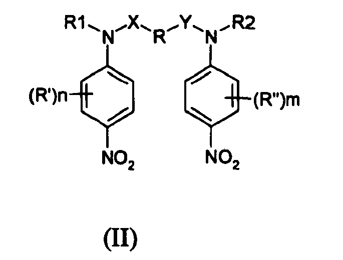 dans laquelle R₁, R₂, X, Y, R, R', R", n et m sont tels que définis précédemment à l'exception des composés 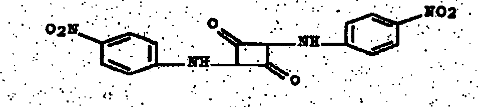 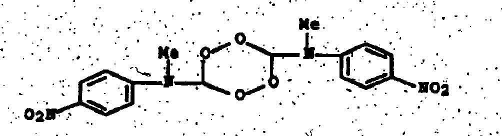 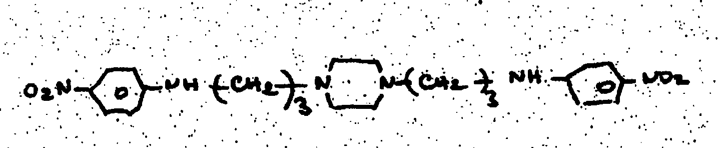 et

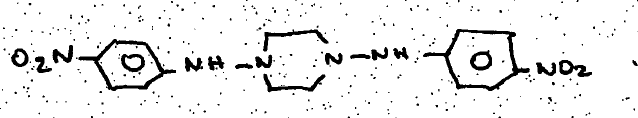

L'invention a aussi pour objet une composition de coloration comprenant dans un milieu approprié à la coloration au moins un coupleur d'oxydation au moins une base d'oxydation du type paraphénylènediamine de formule (I) telle que définie précédemment, cette composition pouvant bien entendu comprendre le composé

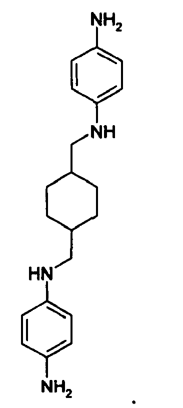

La quantité en paraphénylène-diamine de formule (I) dans la composition de coloration est en général comprise entre 0,0001 % et 20 % en poids par rapport au poids total de la composition, de préférence entre 0,01 % et 10 %.

Parmi les coupleurs d'oxydation, on peut notamment citer les méta-phénylènediamines, les méta-aminophénols, les méta-diphénols, les coupleurs naphtaléniques et les coupleurs hétérocycliques ainsi que leurs sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène (ou résorcinol), le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine, la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Généralement, la quantité du ou des coupleurs d'oxydation est comprise entre 0,0001 et 20%, de préférence entre 0,005 à 6% en poids par rapport au poids total de la composition.

La composition selon l'invention peut également contenir au moins une base d'oxydation additionnelle différente des bases d'oxydation de formule (I).

Les bases d'oxydation peuvent notamment être choisies parmi les para-phénylènediamines, les bis-phénylalkylènediamines, les para--aminophénols, les ortho-aminophénols et les bases hétérocycliques et leurs sels d'addition.

Parmi les para-phénylènediamines, on peut plus particulièrement citer à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, le 2 thiényl paraphénylène diamine, le 2-β hydroxyéthylamino 5-amino toluène, la 3-hydroxy 1-(4' aminophenyl)pyrrolidine, la 6-(4-Amino-phenylamino)-hexan-1-ol et leurs sels d'addition avec un acide.

Parmi les para-phénylènediamines citées ci-dessus, la para-phénylènediamine, la para-toluènediamine, la 2-isopropyl para-phénylènediamine, la 2-β-hydroxyéthyl para-phénylènediamine, la 2-β-hydroxyéthyloxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, la 6-(4-Amino-phénylamino)-hexan-1-ol et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4'-aminophényl) tétraméthylènediamine, la N,N'-bis-(β-hydroxyéthyl) N,N'-bis-(4'-aminophényl) tétraméthylènediamine, la N,N'-bis-(4'-amino-3'-méthylphényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 2-chloro phénol, le 4-amino 3-chloro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, le 4-amino-2,6-dichlorophénol, le 4-amino-6[((5'-amino-2'-hydroxy-3'-méthyl)phényl)méthyl]-2-méthylphénol, le bis(5'-amino-2'-hydroxy)phénylméthane et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, le 5-[(2-hydroxyéthyl)amino]-2-méthylphénol et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1026978 et GB 1153196, comme la 2,5-diamino pyridine, la 2-(4-méthoxyphényl)amino 3-amino pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition avec un acide.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2733749 et DE 19543988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition.

Généralement la concentration de la ou des bases d'oxydation additionnelle est comprise entre 0,0001 et 20%, de préférence entre 0,005 à 6% en poids par rapport au poids total de la composition.

La composition tinctoriale conforme à l'invention peut en outre contenir un ou plusieurs colorants directs pouvant notamment être choisi parmi les colorants nitrés de la série benzénique, neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs méthiniques, azométhiniques, triarylméthaniques, indoaminiques et les colorants directs naturels. De préférence, la composition selon l'invention comprend au moins un colorant choisi parmi les colorants directs cationiques et les colorants directs naturels.

Parmi les colorants directs cationiques utilisables selon l'invention, on peut citer les colorants directs azoïques cationiques décrits dans les demandes de brevets WO 95/15144, WO-95/01772 et EP-714954.

Parmi ces composés, on peut tout particulièrement citer les colorants suivants :
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono) méthyl]-pyridinium.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs représentent de préférence de 0,001 à 20% en poids environ du poids total de la composition prête à l'emploi et encore plus préférentiellement de 0,005 à 10% en poids environ.

Le milieu approprié pour la coloration est avantageusement un milieu cosmétique approprié constitué par de l'eau ou par un mélange d'eau et d'au moins un solvant organique tel que, par exemple, les alcools inférieurs en C₁-C₄, ramifiés ou non, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, le glycérol ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

Avantageusement, la composition de coloration comprend au moins un adjuvant cosmétique choisi parmi les agents anti-oxydants, les agents de pénétration, les agents séquestrants, les parfums, les tampons, les agents dispersants, les tensioactifs, les agents conditionneurs, les agents filmogènes, les polymères, les céramides, les agents conservateurs, les agents nacrants ou opacifiants, les vitamines ou provitamines.

Les adjuvants ci-dessus sont en général présents en quantité comprise pour chacun d'eux entre 0,01 et 20 % en poids par rapport au poids de la composition.

Le pH de la composition conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en coloration des fibres kératiniques ou bien encore à l'aide de systèmes tampons classiques.

Parmi les agents acidifiants, on peut citer, à titre d'exemple, les acides minéraux ou organiques autres que les diacides carboxyliques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule : 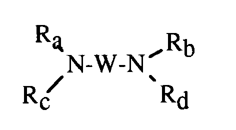 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

Bien entendu, l'homme de l'art veillera à choisir le ou les adjuvants, précurseurs de colorants d'oxydations additionnels, coupleurs d'oxydation, colorants directs de manière telle que les propriétés avantageuses attachées intrinsèquement à la composition de coloration d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une coloration des fibres kératiniques, et notamment des cheveux humains.

Un autre objet de la présente demande concerne un procédé de coloration des fibres kératiniques dans lequel on applique sur les fibres la composition de l'invention telle que définie précédemment, et on révèle la couleur à l'aide d'un agent oxydant. La couleur peut être révélée à pH acide, neutre ou alcalin. L'agent oxydant peut être ajouté à la composition de l'invention juste au moment de l'emploi. Il peut être mis en oeuvre à partir d'une composition oxydante le contenant, appliquée simultanément ou séquentiellement à la composition de l'invention.

A titre d'agents oxydants, on peut citer le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases, le peroxyde d'hydrogène étant particulièrement préféré.

Selon un mode de réalisation particulier, la composition selon la présente invention est mélangée, de préférence au moment de l'emploi, à une composition contenant, dans un milieu approprié pour la coloration, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

La composition oxydante peut renfermer divers adjuvants utilisés classiquement dans les compositions pour la coloration des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en coloration des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une coloration des fibres kératiniques, notamment humaines et en particulier les cheveux humains.

La présente demande concerne également l'utilisation de la composition cosmétique selon l'invention comprenant, dans un milieu approprié pour la coloration, au moins une paraphénylènediamine de formule (1) pour la coloration des fibres kératiniques, de préférence les fibres kératiniques humaines telles que les cheveux.

L'invention a aussi pour objet un dispositif à plusieurs compartiments ou "kit" de coloration dans lequel un premier compartiment renferme une composition tinctoriale contenant une paraphénylènediamine de formule (I) et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

### EXEMPLES DE SYNTHESE

### EXEMPLE 1 : Synthèse du tétrachlorhydrate de la N-(4-aminophényl)-N-[(3-{[(4-aminophényl)amino]méthyl}cyclohexvl)méthyl]amine (2)

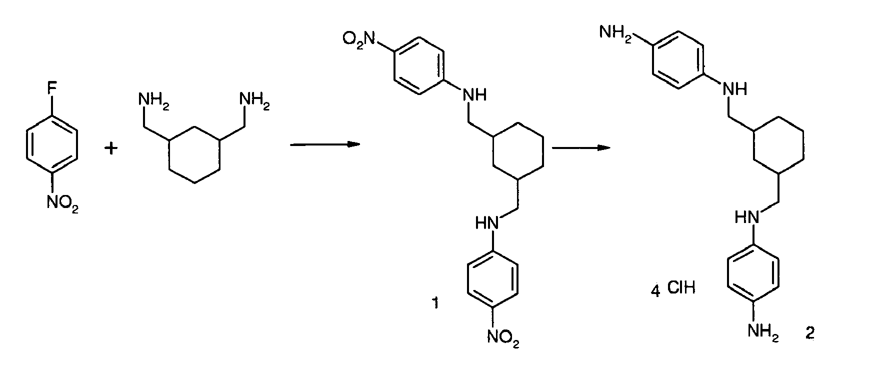

### Etape 1 : synthèse du 4-nitro-N-[(3-{[(4-nitrophényl)amino]méthyl}cyclohexyl) méthyl]aniline (1)

0,83 g de para-fluoronitrobenzène (2éq.) est dissous dans 5 ml de DMSO. 1,2 équivalent de 1,3-bis (aminométhyl)cyclohexane et 4 équivalents de triéthylamine sont ajoutés à la solution. Le milieu réactionnel est porté à 60°C pendant 5 heures. Le mélange est ensuite versé sur de la glace pilée, un précipité se forme. Ce dernier est filtré, lavé à l'eau, puis séché.

### Etape 2: synthèse du tétrachlorhydrate de la N-(4-aminophenyl)-N-[(3-{[(4-aminophenyl)amino]méthyl}cyclohexyl)méthyl]amine (2)

Dans un hydrogénateur, le composé nitré 1 est solubilisé dans 500 ml d'éthanol. 10% de palladium sur charbon (50% humide) sont ajoutés et l'hydrogénateur est chargé en hydrogène. Après 1h30 de réaction, le palladium est filtré et 20 ml d'éthanol chlorhydrique 3M puis 300 ml d'éther isopropylique sont ajoutés au filtrat. Le précipité obtenu est filtré et recristallisé dans l'éthanol chlorhydrique.

Les spectres RMN du proton et de masse sont conformes à la structure attendue du produit.

### EXEMPLE 2 : Synthèse de l'hexachlorhydrate de la N-[3-(4-{3-[(4-aminophenyl)amino]propyl} pipérazin-1-yl)propyl]benzène-1,4-diamine (4)

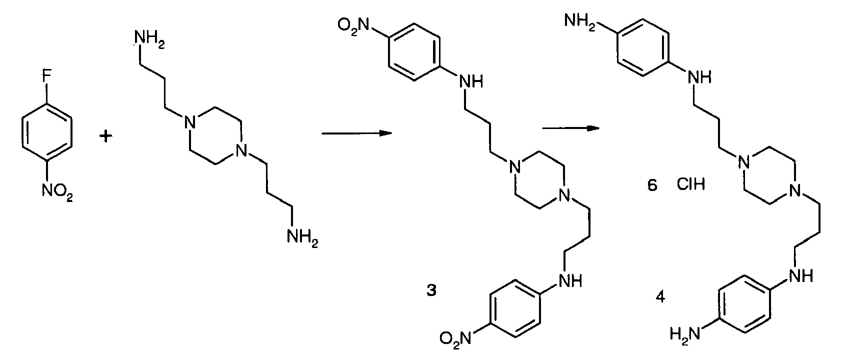

### Etape 1 : synthèse de la 4-nitro-N-[3-(4-{3-[(4-nitrophényl)aminol]propyl} pipérazin-1-yl)propyl]aniline (3)

5 g de para-fluoronitrobenzène (2éq.) sont dissous dans 5 ml de DMSO. 1 équivalent de 1,4-bis(3-aminopropyl)-pipérazine et 2,1 équivalents de triéthylamine sont ajoutés à la solution. Le milieu réactionnel est porté à 60°C pendant 24 heures. Le mélange est ensuite versé sur de la glace pilée, un précipité se forme. Ce dernier est filtré, lavé à l'eau, puis séché.

### Etape 2 : synthèse de l'hexachlorhydrate de la N-[3-(4-{3-[(4-aminophényl)amino]propyl} pipérazin-1-yl)propyl]benzène-1,4-diamine (4)

Dans un hydrogénateur, le composé nitré 3 est solubilisé dans 500 ml d'éthanol. 10% de palladium sur charbon (50% humide) sont ajoutés et l'hydrogénateur est chargé en hydrogène. Après 1h30 de réaction, le palladium est filtré et 20 ml d'éthanol chlorhydrique 3M puis 300 ml d'éther isopropylique sont ajoutés au filtrat. Le précipité obtenu est filtré et recristallisé dans l'éthanol chlorhydrique.

Les spectres RMN du proton et de masse sont conformes à la structure attendue du produit.

### EXEMPLE 3 : Synthèse du tétrachlorhydrate de la N-[(4-{[(4-aminophényl)amino] méthyl}cyclohexyl)méthyl]benzène-1,4-diamine (6)

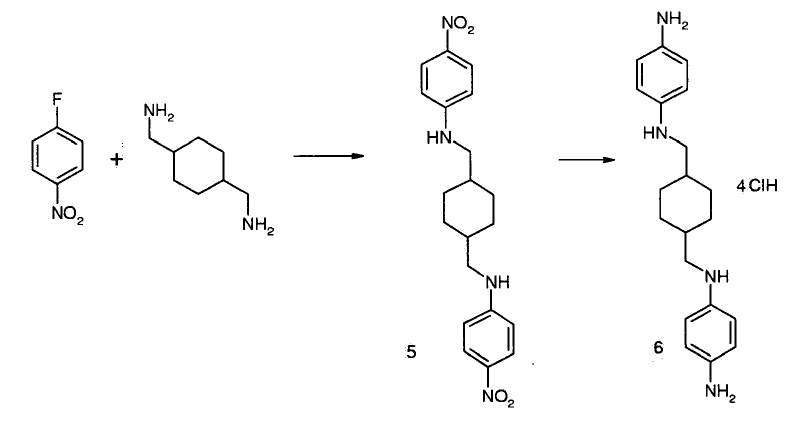

### Etape 1 : synthèse du 4-nitro-N-[(4-{[(4-nitrophényl)amino]méthyl}cyclohexyl)méthyl]aniline (5)

1,653 g de para-fluoronitrobenzène (2éq.) sont dissous dans 5 ml de DMSO. 1,2 équivalents de 1,4-bis (aminométhyl)cyclohexane et 4 équivalents de triéthylamine sont ajoutés à la solution. Le milieu réactionnel est porté à 60°C pendant 7 heures. Le mélange est ensuite versé sur de la glace pilée, un précipité se forme. Ce dernier est filtré, lavé à l'eau, puis séché.

### Etape 2: synthèse du tétrachlorhydrate de la N-[(4-{[(4-aminophényl)amino]méthyl} cyclohexyl)méthyl]benzène-1,4-diamine (6)

Dans un hydrogénateur, 1,2 g de 4-nitro-N-[(4-{[(4-nitrophényl)amino]méthyl}cyclohexyl)méthyl]aniline sont solubilisés dans 500 ml d'éthanol. 0,5 g de palladium sur charbon (50% humide) sont ajoutés et l'hydrogénateur est chargé en hydrogène. Après 1h30 de réaction, le palladium est filtré et 15 équivalents d'éthanol chlorhydrique 3M puis 300 ml d'éther isopropylique sont ajoutés au filtrat. Le précipité obtenu est filtré.

Les spectres RMN du proton et de masse sont conformes à la structure attendue du produit.

### EXEMPLE 4 : Synthèse du tétrachlorhydrate de la N-(4-aminophényl)-N-{3-[(4-aminophényl)amino]cyclohexyl}amine (8)

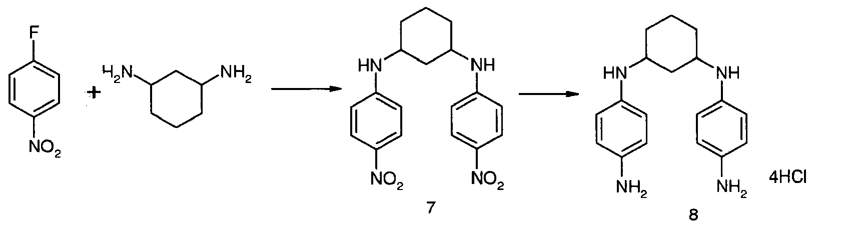

### Etape 1 : synthèse du N-(4-nitrophényl)-N-{3-[(4-nitrophényl)amino] cyclohexyl} amine (7)

4 g de parafluoronitrobenzène (2éq.) sont dissous dans 13 ml de NMP. 1,2 équivalents de cyclohexane-1,3-diamine et 2 équivalents de triméthylamine sont ajoutés à la solution. Le milieu réactionnel est chauffé au micro-ondes (conditions : 200°C pendant 15 minutes, 7 bars). Le mélange est ensuite versé sur 50ml d'acétate d'éthyle, on ajoute 75 ml d'heptane, on observe un dépôt huileux. On récupère l'huile qui précipite dans l'eau glacée. Cette dernière est filtrée, lavée à l'eau, puis séchée.

Les spectres RMN du proton et de masse sont conformes à la structure attendue du produit.

### Etape 2: Synthèse du tétrachlorhydrate de la N-(4-aminophényl)-N-{3-[(4-aminophényl)amino]cyclohexyl}amine (8)

Le composé 7 obtenu lors de l'étape précédente est réduit avec un mélange zinc/chlorure d'ammonium/eau/éthanol bouillant. L'amine résultante est isolée sous forme de tétrachlorhydrate.

### Exemples de teinture

### Exemples 1 à 14 : Composition tinctoriale à partir du tétrachlorhydrate de la N-(4-aminophényl)-N-[(3-{[(4-aminophényl)amino] méthyl}cyclohexyl)méthyl] amine (2)

### Exemples 1 à 7 : Coloration en milieu acide

Les compositions tinctoriales suivantes sont préparées :

| Exemple | 1 | 2 | 3 | 4 | 5 | 6 | 7 |
|---|---|---|---|---|---|---|---|
| tétrachlorhydrate de la N-(4-aminophényl)-N-[(3-{[(4-amino phényl)amino]méthyl}cyclohexyl) méthyl]amine (2) | 10⁻³ mole | 10⁻³ mole | 10⁻³ mole | 10⁻³ mole | 10⁻³ mole | 10⁻³ mole | 10⁻³ mole |
| Benzène-1,3-diol | 10⁻³ mole | | | | | | |
| 5-Amino-2-méthyl-phénol | | 10⁻³ mole | | | | | |
| 1H-Indol-6-ol | | | 10⁻³ mole | | | | |
| 2-Amino-pyridin-3-ol | | | | 10⁻³ mole | | | |
| 3,6-Diméthyl-1H-pyrazolo[5,1-c][1,2 ,4]triazole | | | | | 10⁻³ mole | | |
| chlorhydrate de 2-(2,4-Diamino-phénoxy)-éthanol, | | | | | | 10⁻³ mole | |
| chlorhydrate de 3-Amino-2-chloro-6-méthyl-phénol, | | | | | | | 10⁻³ mole |
| Support de coloration (1) | (*) | (*) | (*) | (*) | (*) | (*) | (*) |
| Eau déminéralisée q.s.p. | 100g | 100g | 100g | 100g | 100g | 100g | 100g |

| | | | | | | | |
|---|---|---|---|---|---|---|---|
| (*) : support de coloration (1) pH 7 | | | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90% de blancs. Après 30 minutes de pose, les mèches sont rincées, lavées avec un shampooing standard, rincées à nouveau puis séchées.

Les nuances obtenues figurent dans le tableau ci-dessous :

| Exemple | 1 | 2 | 3 | 4 | 5 | 6 | 7 |
|---|---|---|---|---|---|---|---|
| Nuance observée | gris intense | gris intense | brun intense | gris intense | gris intense | violet-bleu intense | gris violet intense |

### Exemples 8 à 14 : coloration en milieu basique

Les compositions tinctoriales suivantes sont préparées :

| Exemple | 8 | 9 | 10 | 11 | 12 | 13 | 14 |
|---|---|---|---|---|---|---|---|
| tétrachlorhydrate de la N-(4-aminophenyl)-N-[(3-{[(4-amino phényl)amino]méthyl}cyclohexyl) méthyl]amine(2) | 10⁻³ mole | 10⁻³ mole | 10⁻³ mole | 10⁻³ mole | 10⁻³ mole | 10⁻³ mole | 10⁻³ mole |
| Benzène-1,3-diol | 10⁻³ mole | | | | | | |
| 5-Amino-2-méthyl-phénol | | 10⁻³ mole | | | | | |
| 1H-indol-6-ol | | | 10⁻³ mole | | | | |
| 2-Amino-pyridin-3-ol | | | | 10⁻³ mole | | | |
| 3,6-Diméthyl-1H-pyrazolo [5,1-c][1,2,4]triazole | | | | | 10⁻³ mole | | |
| chlorhydrate de 2-(2,4-Diamino-phénoxy)-éthanol, | | | | | | 10⁻³ mole | |
| chlorhydrate de 3-Amino-2-chloro-6-méthyl-phénol, | | | | | | | 10⁻³ mole |
| Support de coloration (2) | (*) | (*) | (*) | (*) | (*) | (*) | (*) |
| Eau déminéralisée q.s.p. | 100g | 100g | 100g | 100g | 100g | 1 00g | 100g |

| | | | | | | | |
|---|---|---|---|---|---|---|---|
| (*) : support de coloration (2) pH 9.5 | | | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g MA |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH₃ | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90% de blancs. Après 30 minutes de pose, les mèches sont rincées, lavées avec un shampooing standard, rincées à nouveau puis séchées.

Les nuances obtenues figurent dans le tableau ci-dessous :

| Exemple | 8 | 9 | 10 | 11 | 12 | 13 | 14 |
|---|---|---|---|---|---|---|---|
| Nuance observée | brun rouge intense | violet intense | brun rouge intense | gris rouge intense | rouge intense | gris violet-bleu intense | violet-bleu intense |

### Exemples 15 à 28 : Composition tinctoriale à partir de l'hexachlorhydrate de la N-[3-(4-{3-[(4-aminophenyl)amino]propyl}pipérazin-1-yl)propyl]benzène-1,4-diamine (4)

### Exemples 15 à 21 : Coloration en milieu acide

Les compositions tinctoriales suivantes sont préparées :

| Exemple | 15 | 16 | 17 | 18 | 19 | 20 | 21 |
|---|---|---|---|---|---|---|---|
| hexachlorhydrate de la N-[3-(4-{3-[(4-aminophenyl)amino] propyl}pipérazin-1-yl)propyl]benzène-1,4-diamine (4) | 10⁻³ mole | 10⁻³ mole | 10⁻³ mole | 10⁻³ mole | 10⁻³ mole | 10⁻³ mole | 10⁻³ mole |
| Benzène-1,3-diol | 10⁻³ mole | | | | | | |
| 5-Amino-2-méthyl-phénol | | 10⁻³ mole | | | | | |
| 1H-Indol-6-ol | | | 10⁻³ mole | | | | |
| 2-Amino-pyridin-3-ol | | | | 10⁻³ mole | | | |
| 3,6-Diméthyl-1H-pyrazolo[5,1-c][1,2 ,4]triazole | | | | | 10⁻³ mole | | |
| Chlorhydrate de 2-(2,4-Diamino-phénoxy)-éthanol, | | | | | | 10⁻³ mole | |
| Chlorhydrate de 3-Amino-2-chloro-6-méthyl-phénol, | | | | | | | 10⁻³ mole |
| Support de coloration (1) | (*) | (*) | (*) | (*) | (*) | (*) | (*) |
| Eau déminéralisée q.s.p. | 100g | 100g | 100g | 100g | 100g | 100g | 100g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90% de blancs. Après 30 minutes de pose, les mèches sont rincées, lavées avec un shampooing standard, rincées à nouveau puis séchées.

Les nuances obtenues figurent dans le tableau ci-dessous :

| Exemple | 15 | 16 | 17 | 18 | 19 | 20 | 21 |
|---|---|---|---|---|---|---|---|
| Nuance observée | gris intense | gris violet intense | gris rouge intense | brun rouge intense | gris violet-rouge intense | bleu intense | violet intense |

### Exemples 22 à 28 : coloration en milieu basique

Les compositions tinctoriales suivantes sont préparées :

| Exemple | 22 | 23 | 24 | 25 | 26 | 27 | 28 |
|---|---|---|---|---|---|---|---|
| hexachlorhydrate de la N-[3-(4-{3-[(4-aminophenyl)amino]propyl} yl)propyl]benzène-1,4-diamine (4) | 10⁻³ mole | 10⁻³ mole | 10⁻³ mole | 10⁻³ mole | 10⁻³ mole | 10⁻³ mole | 10⁻³ mole |
| Benzène-1,3-diol | 10⁻³ mole | | | | | | |
| 5-Amino-2-méthyl-phénol | | 10⁻³ mole | | | | | |
| 1H-Indol-6-ol | | | 10⁻³ mole | | | | |
| 2-Amino-pyridin-3-ol | | | | 10⁻³ mole | | | |
| 3,6-Diméthyl-1H-pyrazolo[5,1-c][1,2 ,4]triazole | | | | | 10⁻³ mole | | |
| chlorhydrate de 2-(2,4-Diamino-phénoxy)-éthanol, | | | | | | 10⁻³ mole | |
| chlorhydrate de 3-Amino-2-chloro-6-méthyl-phénol, | | | | | | | 10⁻³ mole |
| Support de coloration (2) | (*) | (*) | (*) | (*) | (*) | (*) | (*) |
| Eau déminéralisée q.s.p. | 100g | 100g | 100g | 100g | 100g | 100g | 100g |

| | | | | | | | |
|---|---|---|---|---|---|---|---|
| (*) : support de coloration (2) tel que défini précédemment | | | | | | | |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90% de blancs. Après 30 minutes de pose, les mèches sont rincées, lavées avec un shampooing standard, rincées à nouveau puis séchées.

Les nuances obtenues figurent dans le tableau ci-dessous :

| Exemple | 22 | 23 | 24 | 25 | 26 | 27 | 28 |
|---|---|---|---|---|---|---|---|
| Nuance observée | brun orangé intense | violet intense | rouge intense | brun rouge intense | intense | violet-bleu intense | violet intense |

## Revendications

1. Para-phénylènediamine de formule (I) suivante et sels d'addition correspondants: 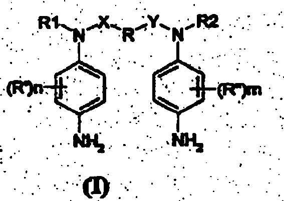 dans laquelle:
• R représente un cycle saturé de 4 à 7 chaînons, contenant éventuellement un ou plusieurs atomes d'azote ou d'oxygène ; ce cycle peut être éventuellement substitué,
• R₁ et R₂ représentent, indépendamment l'un de l'autre,
- un atome d'hydrogène,
- un radical alkyle en C₁-C₆ linéaire ou ramifié,
- un radical alkyle en C₁-C₆ linéaire ou ramifié substitué par un ou plusieurs radicaux hydroxy, alkoxy en C₁₋C₄, amino, monoalkyl amino en C₁-C₄, dialkylamino en C₁₋C₄,
• X et Y représentent, indépendamment l'un de l'autre, un radical alkylène en C₁-C₁₀ linéaire ou ramifié ou une liaison covalente,
• R' et R" représentent, indépendamment l'un de l'autre, un radical alkyle en C₁-C₆, un radical alcoxy en C₁-C₆, un radical hydroxy-alcoxy en C₁-C₆, un radical alcoxy(C₁-C₆)alkyle en C₁-C₆, un radical mono ou poly-hydroxy alkyle en C₁-C₆,
• n et m représentent, indépendamment l'un de l'autre, un entier compris entre 0 et 4 ;
à l'exception des composés suivants :

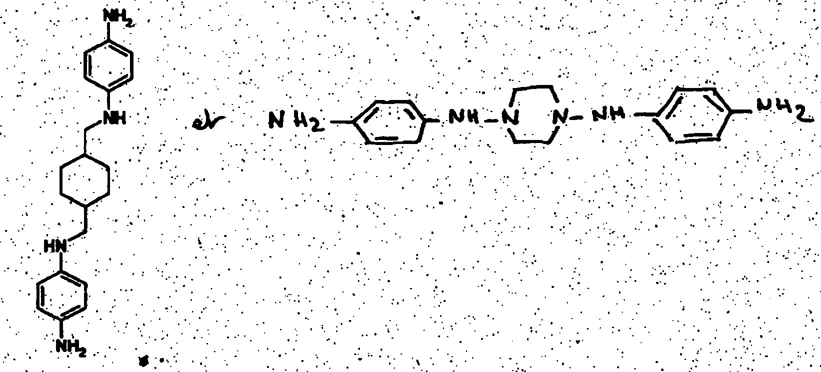

2. Para-phénylènediamine selon la revendication 1 dans laquelle R est un radical cyclique saturé contenant éventuellement un atome d'azote.

3. Para-phénylènediamine selon la revendication 1 ou 2 dans laquelle R est un radical cyclique substitué par un radical alkyle en C₁-C₄.

4. Para-phénylènediamine selon la revendication 1, 2 ou dans laquelle R₁ et R₂ représentent indépendamment un atome d'hydrogène ou un groupe alkyle en C₁-C₄ pouvant être substitué.

5. Para-phénylènediamine selon l'une quelconque des revendications 1 à 4 dans laquelle X et Y représentent indépendamment un radical alkylène en C₁-C₃ ou une liaison covalente.

6. Para-phénylènediamine selon l'une quelconque des revendications 1 à 5 dans laquelle n et m représentent indépendamment 0 ou 1.

7. Para-phénylènediamine selon l'une quelconque des revendications 1 à 6 dans laquelles les sels d'addition avec un acide des para-phénylènediamines secondaires de formule générale (I) sont choisis parmi l'acide chlorhydrique, l'acide bromhydrique, l'acide sulfurique, l'acide citrique, l'acide succinique, l'acide tartrique, l'acide lactique, l'acide para-toluènesulfonique, l'acide benzènesulfonique, l'acide phosphorique et l'acide acétique, ces composés pouvant éventuellement être sous forme de solvates.

8. Par-phénylènediamine selon l'une quelconque des revendications 1 à 7 choisie parmi :

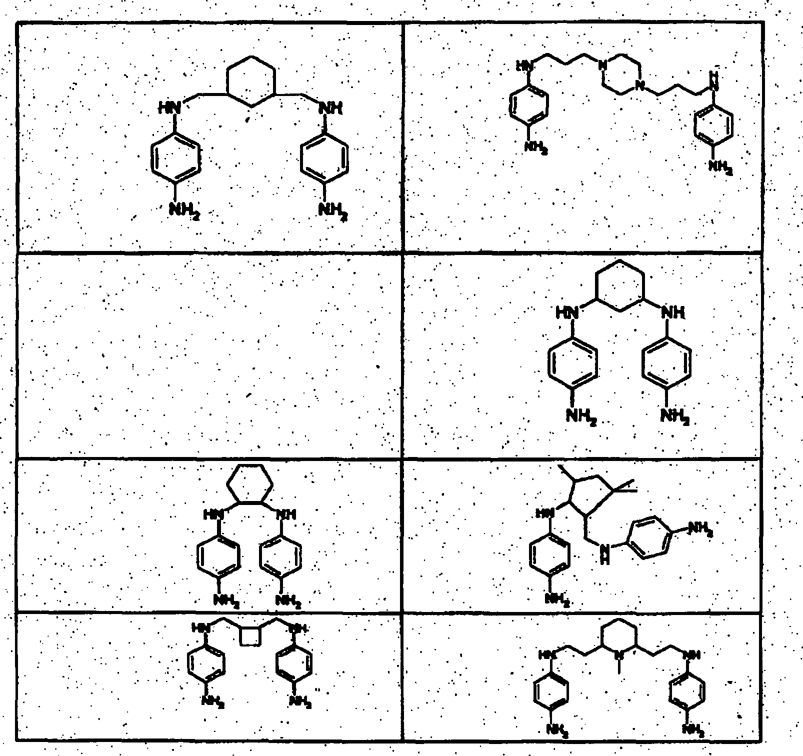

9. Composition de coloration comprenant, dans un milieu approprié à la coloration, au moins une base d'oxydation paraphénylènediamine de formule (I) 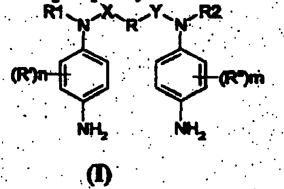 dans laquelle :
• R représente un cycle saturé de 4 à 7 chaînons, contenant éventuellement un ou plusieurs atomes d'azote ou d'oxygène; ce cycle peut être éventuellement substitué,
• R₁ et R₂ représentent, indépendamment l'un de l'autre,
- un atome d'hydogène,
- un radical alkyle en C₁-C₆ linéaire ou ramifié - un radical alkyle en C₁-C₆ linéaire ou ramifié substitué par un ou plusieurs radicaux hydroxy, alkoxy en C₁₋C₄, amino, monoalkyl amino en C₁-C₄, dialkylamino en C₁₋C₄,
• X et Y représentent, indépendamment l'un de l'autre, un radical alkylène en C₁-C₁₀ linéaire ou ramifié ou une liaison covalente,
• R' et R" représentent, indépendamment l'un de l'autre, un radical alkyle en C₁-C₆, un radical alcoxy en C₁-C₆, un radical hydroxy-alcoxy en C₁-C₆, un radical alcoxy(C₁-C₆)alkyle en C₁-C₆, un radical mono ou poly-hydroxy alkyle en C₁-C₆,
• n et m représentent, indépendamment l'un de l'autre, un entier compris entre 0 et 4, et an moins un coupleur d'oxydation.

10. Composition selon la revendication 9 dans laquelle la quantité en para-phénylènediamine de formule (I) est comprise entre 0,001% et 20% en poids par rapport au poids total de la composition, de préférence entre 0,01% et 10%.

11. Composition selon la revendication 9 dans laquelle le coupleur d'oxydation est choisi parmi les méta-phénylènediamines, les méta-aminophénols, les méta-diphénols, les coupleurs naphtaléniques et les coupleurs hétérocycliques ainsi que leurs sels d'addition.

12. Composition selon l'une quelconque des revendications 9 à 11 comprenant de plus au moins une base d'oxydation additionnelle différente des bases d'oxydation de formule (I).

13. Composition selon la revendication 12 dans laquelle les bases d'oxydation peuvent être choisies parmi les para-phénylènediamines, les bis-phénylalkylènediamines, les para-aminophénols, les ortho-aminophénols et les bases hétérocycliques et leurs sels d'addition.

14. Composition selon l'une quelconque des revendications 9 à 12 comprenant au moins un colorant direct.

15. Composition selon l'une quelconque des revendication 9 à 14 dans laquelle le milieu approprié pour la coloration est constitué par de l'eau contenant éventuellement au moins un solvant organique.

16. Composition selon la revendication 15 dans laquelle le solvant organique est choisi parmi les alcools inférieurs en C₁-C₄, ramifiés ou non, ainsi que les alcools aromatiques et leurs mélanges.

17. Composition selon l'une quelconque des revendications 9 à 16 comprenant au moins un adjuvant cosmétique choisi parmi les agents anti-oxydants, les agents de pénétration, le agents séquestrants, les parfums, les tampons, les agents dispersants, les tensioactifs, les agents conditionneurs, les agents filmogènes, les polymères, les céramides, les agents conservateurs, les agents nacrants ou opacifiants, les vitamines ou provitamines.

18. Composition selon la revendication 17 dans laquelle la quantité en adjuvant cosmétique est comprise pour chacun d'eux entre 0,01 et 20% en poids par rapport au poids total de la composition.

19. Composition selon l'une quelconque des revendication précédentes comprenant un agent oxydant.

20. Procédé de coloration des fibres kératiniques **caractérisée par le fait qu'**on applique sur les fibres au moins une composition selon l'une quelconque des revendications 9 à 18 en présence d'un agent oxydant pendant une durée suffisante pour développer la coloration désirée.

21. Dispositif à plusieurs compartiments dans lequel un premier compartiment contient une compostion tinctoriale pour la coloration des fibres kératiniques telle que définie à l'une quelconque des revendications 9 à 18 et un deuxième compartiment contient un agent oxydant.

22. Composé nitro de formule (II) 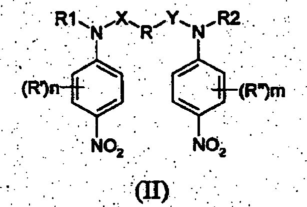 dans laquelle R₁, R₂, X, Y, R, R', R", n, m sont tels que définis aux revendications 1 à 8 à l'exception des composés 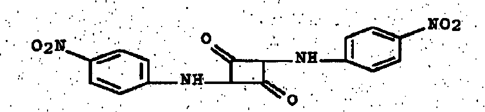 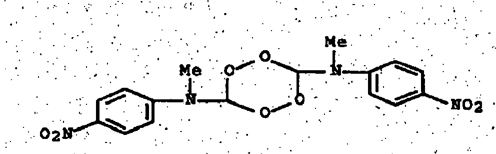 et 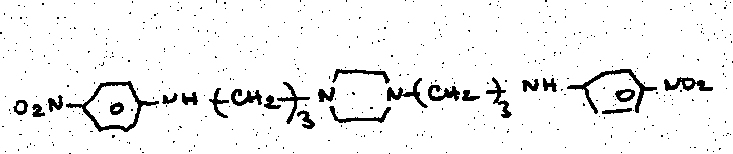 et

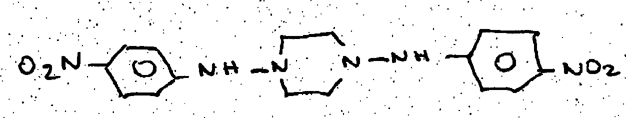

23. Procédé de préparation des para-phénylènediamines de formule (I) par réduction des composés nitrés de formule (II): 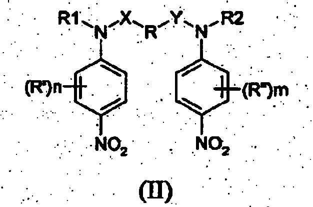 dans laquelle R₁, R₂, X, Y, R, R', R", n, m sont tels que définis aux revendications 1 à 8.

## Claims

1. Para-phenylenediamine of the following formula (I) and corresponding salts of addition: 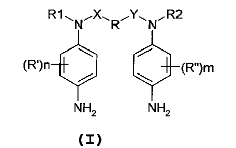 in which:
• R represents a saturated ring with 4 to 7 ring members, optionally containing one or more nitrogen or oxygen atoms; this ring can optionally be substituted,
• R₁ and R₂ represent, independently of one another,
- a hydrogen atom,
- a linear or branched C₁-C₆ alkyl radical,
- a linear or branched C₁-C₆ alkyl radical substituted by one or more hydroxy, C₁-C₄ alkoxy, amino, C₁-C₄ monoalkylamino, or C₁-C₄ dialkylamino radicals,
• X and Y represent, independently of one another, a linear or branched C₁-C₁₀ alkylene radical or a covalent bond,
• R' and R" represent, independently of one another, a C₁-C₆ alkyl radical, a C₁-C₆ alkoxy radical, a C₁-C₆ hydroxy-alkoxy radical, a C₁-C₆ alkoxy(C₁-C₆)alkyl radical, a C₁-C₆ mono- or poly-hydroxy alkyl radical,
• n and m represent, independently of one another, an integer between 0 and 4;
apart from the following compounds:

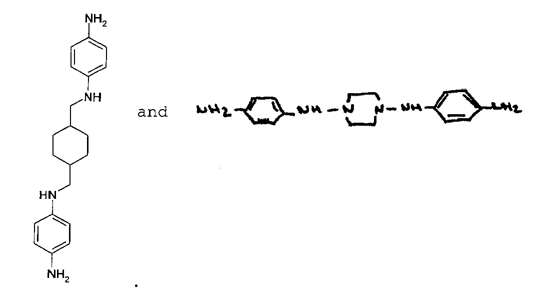

2. Para-phenylenediamine according to Claim 1, **characterized in that** R is a saturated cyclic radical optionally containing a nitrogen atom.

3. Para-phenylenediamine according to Claim 1 or 2, **characterized in that** R is a cyclic radical substituted by a C₁-C₄ alkyl radical.

4. Para-phenylenediamine according to Claim 1, 2 or 3, **characterized in that** R₁ and R₂ represent, independently, a hydrogen atom or a C₁-C₄ alkyl group which may be substituted.

5. Para-phenylenediamine according to any one of Claims 1 to 4, **characterized in that** X and Y represent, independently, a C₁-C₃ alkylene radical or a covalent bond.

6. Para-phenylenediamine according to any one of Claims 1 to 5, **characterized in that** n and m represent, independently, 0 or 1.

7. Para-phenylenediamine according to any one of Claims 1 to 6, **characterized in that** the salts of addition with an acid, of the secondary para-phenylenediamines of general formula (I) are selected from hydrochloric acid, hydrobromic acid, sulphuric acid, citric acid, succinic acid, tartaric acid, lactic acid, para-toluene-sulphonic acid, benzene-sulphonic acid, phosphoric acid and acetic acid, these compounds optionally being in the form of solvates.

8. Para-phenylenediamine according to any one of Claims 1 to 7 selected from:

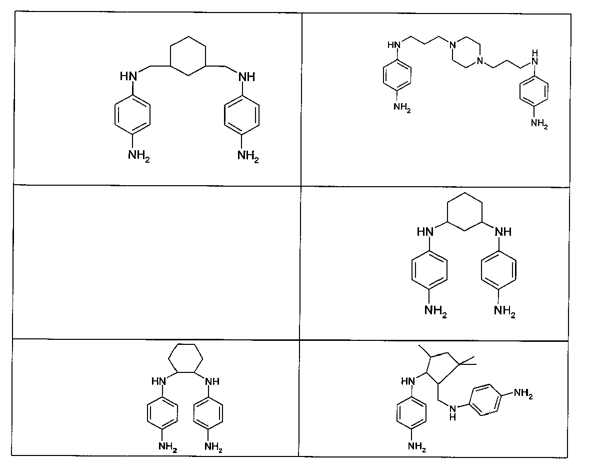

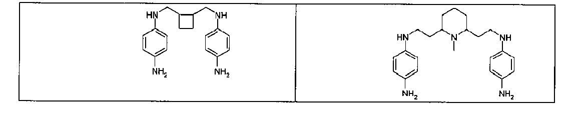

9. Dyeing composition comprising, in a medium that is suitable for dyeing, at least one para-phenylenediamine oxidation base of formula (I) 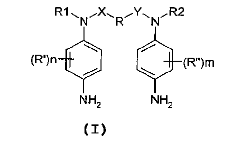 in which:
• R represents a saturated ring with 4 to 7 ring members, optionally containing one or more nitrogen or oxygen atoms; this ring can optionally be substituted,
• R₁ and R₂ represent, independently of one another,
- a hydrogen atom,
- a linear or branched C₁-C₆ alkyl radical,
- a linear or branched C₁-C₆ alkyl radical substituted by one or more hydroxy, C₁-C₄ alkoxy, amino, C₁-C₄ monoalkylamino, or C₁-C₄ dialkylamino radicals,
• X and Y represent, independently of one another, a linear or branched C₁-C₁₀ alkylene radical or a covalent bond,
• R' and R" represent, independently of one another, a C₁-C₆ alkyl radical, a C₁-C₆ alkoxy radical, a C₁-C₆ hydroxy-alkoxy radical, a C₁-C₆ alkoxy(C₁-C₆)alkyl radical, a C₁-C₆ mono- or poly-hydroxy alkyl radical,
• n and m represent, independently of one another, an integer between 0 and 4, and at least one oxidation coupler.

10. Composition according to Claim 9, **characterized in that** the amount of para-phenylenediamine of formula (I) is between 0.0001% and 20 wt.% relative to the total weight of the composition, preferably between 0.01% and 10%.

11. Composition according to Claim 9, **characterized in that** the oxidation coupler is selected from the meta-phenylenediamines, meta-aminophenols, meta-diphenols, naphthalenic couplers and heterocyclic couplers as well as their salts of addition.

12. Composition according to any one of Claims 9 to 11 additionally containing at least one additional oxidation base different from the oxidation bases of formula (I).

13. Composition according to Claim 12, **characterized in that** the oxidation bases can be selected from the para-phenylenediamines, the bis-phenylalkylenediamines, the para-aminophenols, the ortho-aminophenols and the heterocyclic bases and their salts of addition.

14. Composition according to any one of Claims 9 to 12 containing at least one direct dye.

15. Composition according to any one of Claims 9 to 14, **characterized in that** the medium suitable for dyeing comprises water optionally containing at least one organic solvent.

16. Composition according to Claim 15, **characterized in that** the organic solvent is selected from the linear or branched C₁-C₄ lower alcohols, as well as the aromatic alcohols and mixtures thereof.

17. Composition according to any one of Claims 9 to 16 containing at least one cosmetic additive selected from the antioxidants, penetrants, sequestering agents, perfumes, buffers, dispersants, surfactants, conditioners, film-forming agents, polymers, ceramides, preservatives, lustre agents or opacifiers, vitamins or provitamins.

18. Composition according to Claim 17, **characterized in that** the amount of cosmetic additive is, for each of them, between 0.01 and 20 wt.% relative to the total weight of the composition.

19. Composition according to any one of the preceding claims containing an oxidizing agent.

20. Method of dyeing of keratin fibres, **characterized in that** at least one composition according to any one of Claims 9 to 18 is applied to the fibres in the presence of an oxidizing agent for a sufficient time for development of the desired coloration.

21. Multi-compartment kit in which a first compartment contains a dyeing composition for the colouring of keratin fibres as defined in any one of Claims 9 to 18 and a second compartment contains an oxidizing agent.

22. Nitro compound of formula (II) 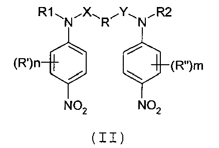 in which R₁, R₂, X, Y, R, R', R", n, m are as defined in Claims 1 to 8 with the exception of the compounds 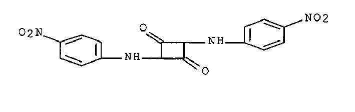 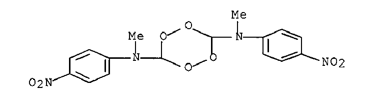 and 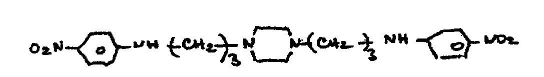 and

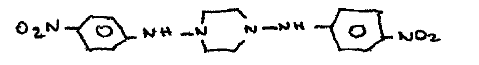

23. Method of production of the para-phenylenediamines of formula (I) by reduction of the nitrogen-containing compounds of formula (II): 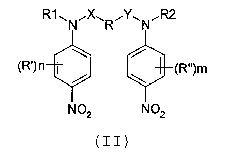 in which R₁, R₂, X, Y, R, R', R", n, m are as defined in Claims 1 to 8.

## Patentansprüche

1. para-Phenylendiamin der folgenden Formel (I) und die zugehörigen Additionssalze: 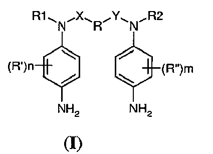 in der Formel:
■ R bedeutet einen 4- bis 7-gliedrigen, gesättigten Ring, der gegebenenfalls ein oder mehrere Stickstoffatome oder Sauerstoffatome enthält; wobei der Ring gegebenenfalls substituiert sein kann;
■ R₁ und R₂ bedeuten unabhängig voneinander:
- ein Wasserstoffatom,
- eine geradkettige oder verzweigte C₁₋₆-Alkylgruppe,
- eine geradkettige oder verzweigte C₁₋₆-Alkylgruppe, die mit einer oder mehreren Gruppen Hydroxy, C₁₋₄-Alkoxy, Amino, Monoalkyl(C₁₋₄)amino, Dialkyl(C₁₋₄)amino substituiert ist,
■ X und Y bedeuten unabhängig voneinander eine geradkettige oder verzweigte C₁₋₁₀-Alkylengruppe oder eine kovalente Bindung;
■ R' und R" bedeuten unabhängig voneinander eine C₁₋₆-Alkylgruppe, eine C₁₋₆-Alkoxygruppe, eine Hydroxyalkoxy(C₁₋₆)-gruppe, eine Alkoxy(C₁₋₆)alkyl(C₁₋₆)gruppe, eine Mono- oder Polyhydroxyalkyl(C₁₋₆)gruppe;
■ n und m bedeuten unabhängig voneinander eine ganze Zahl im Bereich von 0 bis 4;
wobei die folgenden Verbindungen ausgenommen sind:

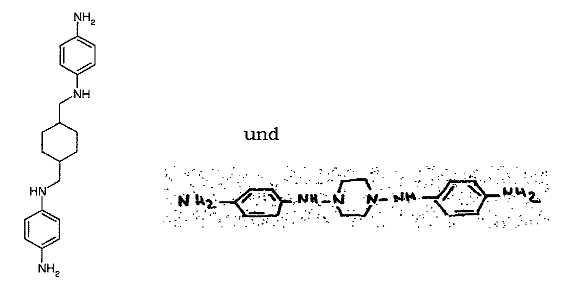

2. para-Phenylendiamin nach Anspruch 1, wobei R eine gesättigte cyclische Gruppe ist, die gegebenenfalls ein Stickstoffatom enthält.

3. para-Phenylendiamin nach Anspruch 1 oder 2, wobei R eine cyclische Gruppe ist, die mit einer C₁₋₄-Alkylgruppe substituiert ist.

4. para-Phenylendiamin nach Anspruch 1, 2 oder 3, wobei R₁ und R₂ unabhängig voneinander ein Wasserstoffatom oder eine C₁₋₄-Alkylgruppe bedeuten, die substituiert sein kann.

5. para-Phenylendiamin nach einem der Ansprüche 1 bis 4, wobei X und Y unabhängig voneinander eine C₁₋₃-Alkylengruppe oder eine kovalente Bindung bedeuten.

6. para-Phenylendiamin nach einem der Ansprüche 1 bis 5, wobei n und m unabhängig 0 oder 1 bedeuten.

7. para-Phenylendiamin nach einem der Ansprüche 1 bis 6, wobei die Additionssalze der sekundären para-Phenylendiamine der allgemeinen Formel (I) mit einer Säure unter Salzsäure, Bromwasserstoffsäure, Schwefelsäure, Citronensäure, Bernsteinsäure, Weinsäure, Milchsäure, *p*-Toluolsulfonsäure, Benzolsulfonsäure, Phosphorsäure und Essigsäure ausgewählt sind, wobei diese Verbindungen gegebenenfalls in der Form von Solvaten vorliegen.

8. para-Phenylendiamin nach einem der Ansprüche 1 bis 7, das ausgewählt ist unter:

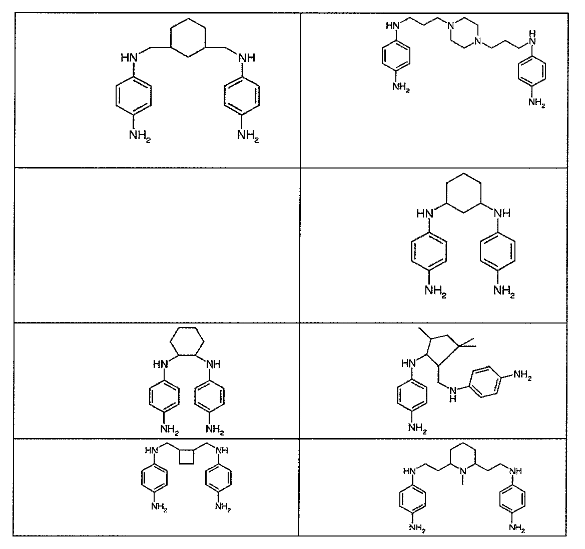

9. Farbmittelzusammensetzung, die in einem zum Färben geeigneten Medium mindestens eine para-Phenylendiamin-Oxidationsbase der Formel (I): 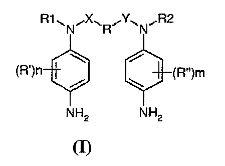 in der Formel:
■ R bedeutet einen 4- bis 7-gliedrigen, gesättigten Ring, der gegebenenfalls ein oder mehrere Stickstoffatome oder Sauerstoffatome enthält; wobei der Ring gegebenenfalls substituiert sein kann;
■ R₁ und R₂ bedeuten unabhängig voneinander:
- ein Wasserstoffatom,
- eine geradkettige oder verzweigte C₁₋₆-Alkylgruppe,
- eine geradkettige oder verzweigte C₁₋₆-Alkylgruppe, die mit einer oder mehreren Gruppen Hydroxy, C₁₋₄-Alkoxy, Amino, Monoalkyl(C₁₋₄)amino, Dialkyl(C₁₋₄)amino substituiert ist,
■ X und Y bedeuten unabhängig voneinander eine geradkettige oder verzweigte C₁₋₁₀-Alkylengruppe oder eine kovalente Bindung;
■ R' und R" bedeuten unabhängig voneinander eine C₁₋₆-Alkylgruppe, eine C₁₋₆-Alkoxygruppe, eine Hydroxyalkoxy(C₁₋₆)-gruppe, eine Alkoxy(C₁₋₆)alkyl(C₁₋₆)gruppe, eine Mono- oder Polyhydroxyalkyl(C₁₋₆)gruppe;
■ n und m bedeuten unabhängig voneinander eine ganze Zahl im Bereich von 0 bis 4;
und mindestens einen oxidativen Kuppler enthält.

10. Zusammensetzung nach Anspruch 9, wobei der Mengenanteil des para-Phenylendiamins der Formel (I) im Bereich von 0,0001 bis 20 Gew.-%, bezogen auf das Gesamtgewicht der Zusammensetzung, und vorzugsweise im Bereich von 0,01 bis 10 Gew.-% liegt.

11. Zusammensetzung nach Anspruch 9, wobei der oxidative Kuppler unter den meta-Phenylendiaminen, meta-Aminophenolen, meta-Dihydroxybenzolen, Naphthalinkupplern und heterocyclischen Kupplern sowie deren Additionssalzen ausgewählt ist.

12. Zusammensetzung nach einem der Ansprüche 9 bis 11, die mindestens eine ergänzende Oxidationsbase enthält, die von den Oxidationsbasen der Formel (I) verschieden sind.

13. Zusammensetzung nach Anspruch 12, wobei die Oxidationsbasen unter den para-Phenylendiaminen, Bisphenylalkylendiaminen, para-Aminophenolen, ortho-Aminophenolen und den heterocyclischen Oxidationsbasen und deren Additionssalzen ausgewählt sind.

14. Zusammensetzung nach einem der Ansprüche 9 bis 12, die mindestens einen Direktfarbstoff enthält.

15. Zusammensetzung nach einem der Ansprüche 9 bis 14, wobei das zum Färben geeignete Medium aus Wasser besteht, das gegebenenfalls mindestens ein organisches Lösmittel enthält.

16. Zusammensetzung nach Anspruch 15, wobei das organische Lösemittel unter den verzweigten oder unverzweigten niederen C₁₋₄-Alkoholen sowie den aromatischen Alkoholen und deren Gemischen ausgewählt ist.

17. Zusammensetzung nach einem der Ansprüche 9 bis 16, die mindestens einen kosmetischen Zusatzstoff enthält, der unter den Antioxidantien, Penetrationsmitteln, Maskierungsmitteln, Parfums, Puffern, Dispergiermitteln, grenzflächenaktiven Stoffen, Konditioniermitteln, Filmbildnern, Polymeren, Ceramiden, Konservierungsmitteln, Perlglanzstoffen oder Trübungsmittel, Vitaminene oder Provitaminen ausgewählt ist.

18. Zusammensetzung nach Anspruch 17, wobei der Mengenanteil jedes kosmetischen Zusatzstoffes im Bereich von 0,01 bis 20 Gew.-%, bezogen auf das Gesamtgewicht der Zusammensetzung, liegt.

19. Zusammensetzung nach einem der vorhergehenden Ansprüche, die ein Oxidationsmittel enthält.

20. Verfahren zum Färben von Keratinfasern, **dadurch gekennzeichnet, dass** auf die Keratinfasern mindestens eine Zusammensetzung nach einem der Ansprüche 9 bis 18 in Gegenwart eines Oxidationsmittels während einer Zeitspanne, die ausreichend ist, um die gewünschte Färbung zu bilden, aufgebracht wird.

21. Vorrichtung mit mehreren Abteilungen, wobei eine erste Abteilung eine Farbmittelzusammensetzung zum Färben von Keratinfasern enthält, wie sie in einem der Ansprüche 9 bis 18 definiert ist, und eine zweite Abteilung ein Oxidationsmittel enthält.

22. Nitro-Verbindung der Formel (II): 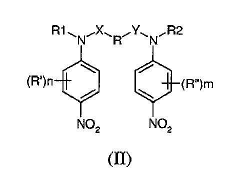 wobei R₁, R₂, X, Y, R, R', R", n und m die in den Ansprüchen 1 bis 8 angegebenen Bedeutungen aufweisen, wobei die folgenden Verbindungen ausgenommen sind: 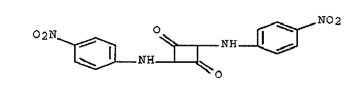 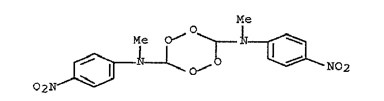 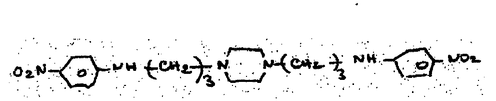 und

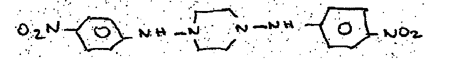

23. Verfahren zur Herstellung von para-Phenylendiaminen der Formel (I) durch Reduktion der nitrierten Verbindungen der Formel (II): 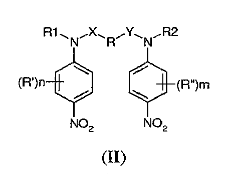 wobei R₁, R₂, X, Y, R, R', R", n und m die in den Ansprüchen 1 bis 8 angegebenen Bedeutungen aufweisen.
